# FASCICULE DE BREVET EUROPEEN

(11) **EP 3 877 006 B1**
(45) Date de publication et mention de la délivrance du brevet: **12.06.2024**
(21) Numéro de dépôt: 19816834.6
(22) Date de dépôt: 30.10.2019
(51) Int. Cl.: A61L 2/08, B65B 55/00

(54) **PROCÉDÉ DE TRAITEMENT POUR LA STÉRILISATION PAR IRRADIATION DE RÉCIPIENTS EN MATIÈRE THERMOPLASTIQUE**
BEHANDLUNGSVERFAHREN ZUR STRAHLENSTERILISATION VON BEHÄLTERN AUS THERMOPLASTISCHEM MATERIAL
TREATMENT METHOD FOR RADIATION STERILISATION OF CONTAINERS MADE FROM THERMOPLASTIC MATERIAL

(30) Priorité: 09.11.2018 FR 1860356
(43) Date de publication de la demande: 15.09.2021
(73) Titulaire: Sidel Participations, 76930 Octeville-sur-Mer (FR)
(72) Inventeur: BERNARD, Véronique, 76930 Octeville-sur-mer (FR); CHOMEL, Nicolas, 76930 Octeville-sur-mer (FR); LE PECHOUR, Anthony, 76930 Octeville-sur-mer (FR)
(74) Mandataire: Sidel Group
(86) Numéro de dépôt international: PCT/FR2019/052577
(87) Numéro de publication internationale: WO 2020/094948

(56) Documents cités:
- EP-A1- 2 316 495
- EP-A1- 3 360 810
- JP-A- H11 114 030
- JP-A- 2007 126 168

## Description

### DOMAINE TECHNIQUE DE L'INVENTION

L'invention concerne un procédé de traitement pour la stérilisation par irradiation de récipients en matière thermoplastique.

L'invention concerne plus particulièrement un procédé de traitement pour la stérilisation par irradiation de récipients en matière thermoplastique, tels que des bouteilles, présentant un axe principal et comportant un corps muni d'un col et fermé par un fond.

### ÉTAT DE LA TECHNIQUE

On connaît de l'état de la technique différents procédés de stérilisation pour stériliser au moins l'intérieur d'une préforme et/ou d'un récipient en matière thermoplastique.

La fabrication d'un récipient en matière thermoplastique est obtenue à partir d'une préforme chaude, généralement préalablement conditionnée thermiquement dans un four d'une installation de fabrication de récipients avant d'être introduite dans un moule pour y être transformée par soufflage au moyen d'au moins un fluide sous pression, avec ou sans étirage.

On fabrique ainsi différents types de récipients (bouteilles, flacons, pots, etc.) qui sont notamment, mais non exclusivement, destinés à être utilisés pour le conditionnement de produits dans l'industrie agro-alimentaire.

Dans le domaine de la fabrication de récipients pour l'industrie agro-alimentaire, on recherche par tous moyens à réduire les risques de contaminations microbiologiques des récipients par des agents pathogènes, soit des micro-organismes.

C'est la raison pour laquelle, la Demanderesse a déjà proposé de mettre en oeuvre différentes actions pour éliminer des agents pathogènes, tels que les germes (bactéries, moisissures, etc.), qui sont susceptibles d'affecter le produit contenu dans de tels récipients.

On peut en particulier distinguer d'une part les actions visant à détruire les micro-organismes pour stériliser au moins l'intérieur du récipient et, d'autre part, les actions visant plus généralement à prévenir la contamination des récipients par de tels micro-organismes.

Les documents de l'état de la technique FR-2.915.127, WO-03/084818 et EP-2.094.312, auxquels on se reportera pour de plus amples détails, sont cités à titre d'exemples non limitatifs de telles actions.

Le document FR-2.915.127 décrit une installation de fabrication de récipients comportant une enceinte de protection délimitant une zone à l'intérieur de laquelle est agencée une machine de moulage de récipients de type souffleuse qui est alimentée par des moyens de transfert en préformes préalablement conditionnées thermiquement dans un four.

Selon les enseignements de ce document, l'installation comporte un système d'insufflation d'air filtré à l'intérieur de l'enceinte pour y établir notamment une surpression de manière à limiter les risques de contamination tant des préformes en sortie du four que des récipients fabriqués.

Le document WO-03/084818 décrit par exemple un traitement de décontamination par irradiation du col de préformes par un rayonnement de type ultraviolet (UV), avant l'introduction des préformes dans le four.

Le document JP H11 114030 décrit un système de stérilisation des bouteilles en matière thermoplastique avec un faisceau d'électrons.

Le document EP-2.094.312 décrit par exemple un traitement par irradiation avec un rayonnement ultraviolet (UV) mis en oeuvre de manière particulière dans un four pour décontaminer au moins la surface externe de la préforme en cours de conditionnement thermique.

Le document WO-2006/136498 au nom de la Demanderesse décrit par exemple un traitement de décontamination d'une préforme consistant à déposer par condensation un film de buée sensiblement uniforme d'un agent stérilisant sur la paroi interne de la préforme.

Un tel traitement de décontamination par condensation, dit par « voie chimique », donne satisfaction puisque des degrés de décontamination jusqu'à 6Log sont obtenus.

On rappelle que la quantité de micro-organismes est susceptible d'être dénombrée par comptage après notamment des opérations de lavage, de filtration et de mise en culture.

On détermine ainsi une réduction logarithmique du nombre de micro-organismes par exemple dite de l'ordre de 3Log (ou encore 3D) équivalent à 1000 unités (10³).

Cependant, on recherche des solutions alternatives à la décontamination par voie chimique permettant de ne pas utiliser d'agent stérilisant, comme le peroxyde d'hydrogène (H2O2), mais sans toutefois sacrifier pour autant au résultat obtenu pour la décontamination.

Dans le document WO-2016/120544, la Demanderesse a proposé une solution alternative consistant à procéder à la stérilisation d'un récipient en matière thermoplastique au moyen d'un faisceau d'électrons pulsé et d'un réflecteur mobile.

Pour l'application industrielle d'un tel procédé de traitement pour la stérilisation de récipients, outre la maîtrise technique, l'un des enjeux est aujourd'hui essentiellement d'ordre économique, en raison du coût élevé d'un émetteur utilisé pour obtenir le faisceau d'électrons.

C'est la raison pour laquelle, on recherche des solutions permettant d'optimiser l'utilisation d'un émetteur, de réduire le nombre total d'émetteurs, et cela tout en ayant les mêmes résultats de stérilisation des récipients.

Or, il est nécessaire d'avoir une durée d'irradiation qui soit suffisante lors du traitement pour irradier toutes les surfaces du récipient à stériliser avec une quantité d'électrons permettant d'obtenir une dose létale pour les micro-organismes.

Le procédé de traitement doit également être compatible avec les cadences actuelles de production de récipients qui atteignent par exemple, dans le cas de bouteilles en PET, plus de 60.000 bouteilles par heure.

Le but de l'invention est notamment de proposer un nouveau procédé de traitement permettant de résoudre au moins une partie des inconvénients de l'état de la technique et tout particulièrement de réduire le nombre d'émetteur(s) et d'en optimiser l'utilisation pour chaque type de récipients.

### BREF RESUME DE L'INVENTION

Dans ce but, l'invention propose un procédé de traitement pour la stérilisation par irradiation de récipients en matière thermoplastique présentant un axe principal et comportant un corps muni d'un col et fermé par un fond, dans lequel les récipients formant un flux sont transportés par un système de convoyage suivant un parcours donné, avec un écartement déterminé, dit pas, correspondant à la distance entre les axes de deux récipients consécutifs, et la vitesse de déplacement des récipients formant ledit flux est constante, le procédé de traitement comportant au moins :
- une étape initiale de modification du pas consistant à faire varier sélectivement ledit pas initial entre deux récipients consécutifs pour augmenter la durée d'irradiation de chaque récipient ;
- une étape d'irradiation consistant à irradier chaque récipient du flux depuis l'extérieur avec un faisceau d'électrons émis par au moins un émetteur d'un dispositif de stérilisation qui est agencé sur un tronçon dudit parcours de manière à former une zone d'irradiation des récipients.

Avantageusement, le procédé selon l'invention permet en outre de réduire le nombre d'émetteurs nécessaires grâce à l'augmentation de la durée d'irradiation des récipients résultant de la variation de pas mise en oeuvre.

Selon l'invention, chaque récipient est irradié avec une dose qui est supérieure ou égale à celle de l'art antérieur, c'est-à-dire une dose létale pour les micro-organismes, mais avec un nombre moindre d'émetteurs.

Avantageusement, ladite dose létale est ainsi obtenue avec moins d'émetteur(s) en raison de l'augmentation de la durée d'irradiation qui résulte de la variation du pas réalisée sélectivement de manière à pouvoir réduire la vitesse au moins dans la ou les zones d'irradiation, mais sans toutefois réduire au final la cadence, c'est-à-dire la vitesse moyenne de circulation des récipients.

Avantageusement, la mise en oeuvre d'un procédé de traitement selon l'invention est compatible avec les cadences de fabrication de récipients et donc susceptible de recevoir une application industrielle en intégrant un dispositif de stérilisation pour sa mise en oeuvre au sein d'une installation de fabrication de récipients, tels que des bouteilles en PET.

Avantageusement, la stérilisation du récipient selon le procédé de l'invention est effectuée en irradiant un récipient vide, avant de procéder au remplissage.

De préférence, le procédé selon l'invention est mis en oeuvre dans une installation de fabrication de récipients entre l'unité de moulage (ou souffleuse) et l'unité suivante, tel qu'une unité de remplissage ou d'étiquetage.

Selon les applications, un étiquetage des récipients est en effet susceptible d'être réalisé avant ou après leur remplissage.

Par comparaison avec un procédé de décontamination de préformes par voie chimique selon le document WO-2006/136498 précité, l'invention permet de simplifier grandement la conception d'une installation de fabrication de récipients à partir d'une préforme, en particulier l'unité de moulage (ou souffleuse).

La stérilisation du récipient final (et non de la préforme) permet de s'affranchir de nombreux moyens jusqu'alors mis en oeuvre dans une telle installation de fabrication de récipients, les micro-organismes présents étant détruits lors de l'irradiation du récipient au moyen du faisceau d'électrons, préférentiellement de type pulsé.

Ainsi, il n'est plus nécessaire de mettre en oeuvre des moyens spécifiques (tels que des systèmes d'insufflation, etc.) pour préserver la stérilité d'une préforme postérieurement à son traitement chimique, c'est-à-dire au cours de son conditionnement thermique, de sa transformation par soufflage ou étirage-soufflage en récipient et cela jusqu'au remplissage et à la fermeture du récipient.

Avantageusement, le procédé selon l'invention permet de stériliser simultanément tant l'intérieur que l'extérieur d'un récipient.

Ainsi, les dispositifs de traitement des préformes par irradiation au moyen d'un rayonnement UV sont susceptibles d'être supprimés.

Les systèmes d'insufflation et plus généralement de filtration de l'air participant à l'obtention d'un environnement de fabrication propre sont également susceptibles d'être supprimés.

Avantageusement, la suppression de l'ensemble de tels dispositifs et/ou systèmes permet de réaliser des économies importantes sur le coût d'une installation de fabrication et cela tant à l'acquisition qu'à l'exploitation.

Avantageusement, le procédé selon l'invention est mis en oeuvre dans une installation de fabrication de récipients en aval de l'unité de moulage (ou souffleuse) de sorte que les actions réalisées en amont de l'unité de moulage, notamment en vue de la destruction des micro-organismes et de la prévention des risques de contamination des récipients, peuvent être en tout ou en partie supprimées.

Avantageusement, la conception de l'unité de moulage (ou souffleuse) s'en trouve particulièrement simplifiée et son coût de fabrication comme d'exploitation réduit. En effet, les opérations de nettoyage dites « CIP » (acronyme pour « Clean-in-Place » en anglais) peuvent être supprimées.

Par conséquent, il n'est plus nécessaire de recourir pour l'unité de moulage à l'utilisation de matériaux coûteux, tels que l'inox, choisis pour leur résistance aux agressions chimiques, notamment la corrosion, résultant des produits de nettoyage utilisés lors de telles opérations de « CIP ».

Avantageusement, le traitement de stérilisation par irradiation selon l'invention est susceptible d'être réalisé en combinaison avec les variantes de réalisation permettant d'optimiser l'irradiation en maximisant la dose reçue par chaque récipient.

Avantageusement, on appréciera que l'invention va à l'encontre des enseignements de l'état de la technique consistant, pour préserver la cadence, à multiplier le nombre d'émetteurs le long du parcours jusqu'à atteindre une dose d'irradiation suffisante pour stériliser chaque récipient.

Selon d'autres caractéristiques de l'invention :
- l'étape initiale de modification dudit pas initial consiste à réduire l'écartement entre les récipients dudit flux en réduisant la vitesse des récipients transportés pour obtenir, au moins dans ladite zone d'irradiation, un pas proximal qui est inférieur au pas initial ;
- l'étape initiale de modification dudit pas initial comporte au moins une phase de décélération des récipients pour obtenir ledit pas proximal ;
- ledit procédé comporte une étape finale de modification du pas comportant au moins une phase d'accélération pour faire varier à nouveau l'écartement des récipients afin de rétablir ledit pas initial entre les récipients dudit flux ;
- ledit procédé comporte une étape de division du flux, mise en oeuvre préalablement à l'étape initiale de modification du pas, consistant à diviser le flux de récipients transportés, en au moins :
   - un premier flux de récipients qui sont transportés vers une première zone d'irradiation comportant au moins un premier émetteur apte à émettre un faisceau d'électrons et
   - un deuxième flux de récipients qui sont transportés vers une deuxième zone d'irradiation comportant au moins un deuxième émetteur apte à émettre un faisceau d'électrons ;
- le flux de récipients est divisé à raison d'un récipient sur deux pour former respectivement ledit premier flux et ledit deuxième flux de sorte que l'écartement entre deux récipients consécutifs de l'un ou l'autre desdits premier flux et deuxième flux est alors égal à un pas distal qui est supérieur au pas initial ;
- après la division du flux de récipients, ladite étape initiale de modification du pas initial comporte au moins une phase de décélération pour réduire la vitesse de chacun des récipients constituant l'un desdits au moins premier flux et deuxième flux au moins dans lesdites première zone d'irradiation et deuxième zone d'irradiation associées ;
- après l'étape de division du flux de récipients, ladite étape de modification du pas initial comporte au moins une phase d'accélération pour augmenter sélectivement la vitesse de chacun des récipients desdits premier flux et deuxième flux, respectivement en amont et/ou en aval desdites première zone et deuxième zone d'irradiation ;
- ledit procédé comporte une étape finale de modification du pas consistant, après ladite au moins une étape d'irradiation, à fusionner ensemble les récipients desdits premier flux et deuxième flux pour obtenir un flux de récipients présentant ledit pas initial entre deux récipients consécutifs ;
- ladite étape d'irradiation comporte une première étape d'irradiation consistant à irradier depuis l'extérieur au moins le corps de chaque récipient avec le faisceau d'électrons émis par ledit au moins un émetteur qui présentant un axe principal ;
- lorsque la direction de déplacement suivi par le flux de récipients est orthogonale à l'axe principal des récipients, ledit au moins un émetteur est agencé relativement audit parcours de manière que ledit axe principal de l'émetteur soit coaxial avec l'axe principal du récipient irradié ;
- lorsque la direction de déplacement suivi par le flux de récipients comporte au moins un tronçon incliné relativement à l'axe dudit au moins émetteur de sorte que chaque récipient effectue un déplacement en oblique suivant un mouvement de montée ou de descente, ledit au moins un émetteur est agencé horizontalement pour présenter un angle d'inclinaison de 90° entre son axe principal et l'axe d'un récipient ;
- chaque récipient est entrainé en rotation sur lui-même autour de son axe lors au moins de ladite première étape d'irradiation ;
- ladite étape d'irradiation comporte une deuxième étape d'irradiation consistant à irradier au moins le col de chaque récipient avec un faisceau d'électrons émis par ledit au moins un émetteur qui, présentant un axe principal, est agencé à l'aplomb des récipients suivant ledit parcours ;

Avantageusement, ledit procédé comporte une étape finale de modification du pas consistant, après ladite au moins une étape d'irradiation, à faire varier de nouveau l'écartement des récipients pour rétablir ledit pas initial entre les récipients dudit flux.

Avantageusement, ledit procédé comporte une étape préliminaire de détermination consistant, en fonction du diamètre extérieur maximal d'un récipient, à déterminer une valeur minimale dudit pas proximal pour laquelle tout contact entre deux récipients consécutifs est évité, en particulier lors de l'étape d'irradiation au cours de laquelle chaque récipient est entraîné en rotation sur lui-même.

Avantageusement, chaque récipient est immobile en rotation lors de ladite deuxième étape d'irradiation.

Avantageusement, ledit procédé comporte au moins une étape préliminaire de réglage consistant, lorsque la direction de déplacement suivi par le flux de récipients est orthogonale à l'axe principal des récipients, à positionner ledit au moins un émetteur présentant un axe principal suivant un angle d'inclinaison qui, compris entre l'axe principal de l'émetteur et l'axe du récipient, est déterminé en fonction de la hauteur du récipient pour que le ratio de la hauteur du récipient sur la hauteur de l'émetteur soit proche de 1 .

Avantageusement, ledit au moins un émetteur étant agencé pour présenter un angle d'inclinaison de 90° entre son axe principal et l'axe d'un récipient, ledit procédé comporte au moins une étape préliminaire de paramétrage consistant à paramétrer le système de convoyage pour faire varier au moins la vitesse de déplacement du flux de récipients qui est transportés suivant un parcours comportant un tronçon incliné relativement à l'axe dudit au moins émetteur de sorte que chaque récipient effectue un mouvement de montée ou de descente.

Avantageusement, l'étape d'irradiation pour stériliser les récipients depuis l'extérieur est obtenue au moyen d'un faisceau d'électrons pulsé.

### BREVE DESCRIPTION DES FIGURES

D'autres caractéristiques et avantages de l'invention apparaitront au cours de la lecture de la description détaillée qui va suivre pour la compréhension de laquelle on se reportera aux dessins annexés dans lesquels :

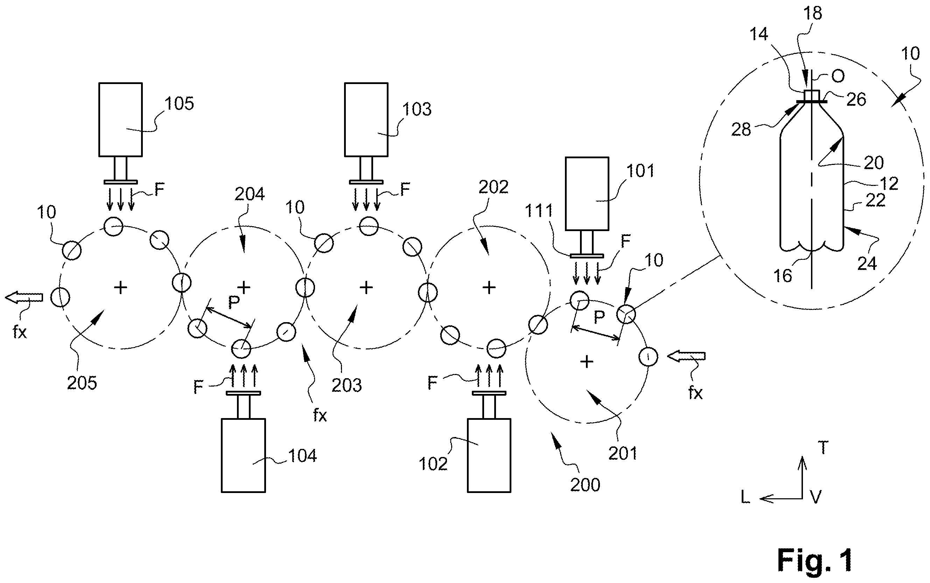

- la figure 1 est une vue de dessus qui représente schématiquement un flux de récipients en matière thermoplastique, par exemple des bouteilles, qui sont transportés avec un pas constant par un système de convoyage suivant un parcours le long duquel sont agencés des émetteurs d'un dispositif de stérilisation destinés à irradier lesdits récipients depuis l'extérieur avec un faisceau d'électrons et qui illustre la mise en oeuvre d'un procédé de traitement pour la stérilisation par irradiation de récipients selon l'état de la technique ;

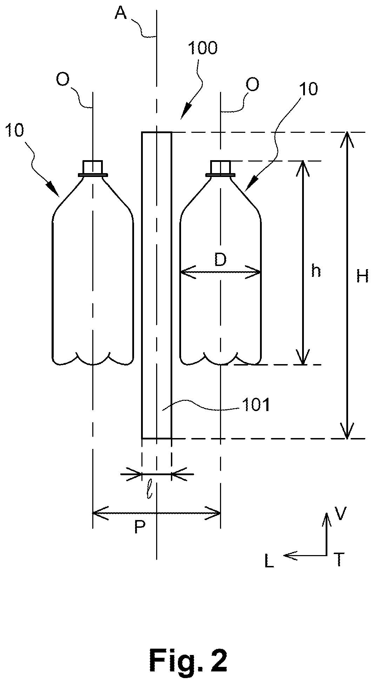

- la figure 2 est une vue de côté qui représente un des émetteurs du dispositif de stérilisation selon la figure 1 et deux récipients consécutifs du flux et qui illustre un tronçon de parcours formant une zone de stérilisation par irradiation des récipients selon l'état de la technique ;

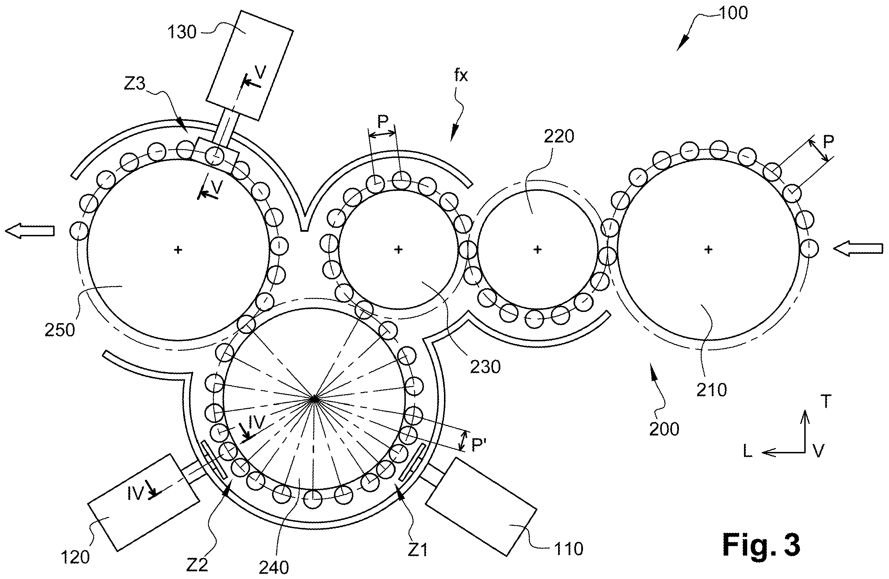

- la figure 3 est une vue de dessus qui, similaire à la figure 1, représente un flux de récipients en matière thermoplastique transportés par un système de convoyage suivant un parcours donné et qui illustre un premier exemple de réalisation pour la mise en oeuvre du procédé de traitement pour la stérilisation par irradiation des récipients selon l'invention dans lequel le pas entre deux récipients consécutifs est temporairement réduit afin d'augmenter la durée d'irradiation des récipients ;

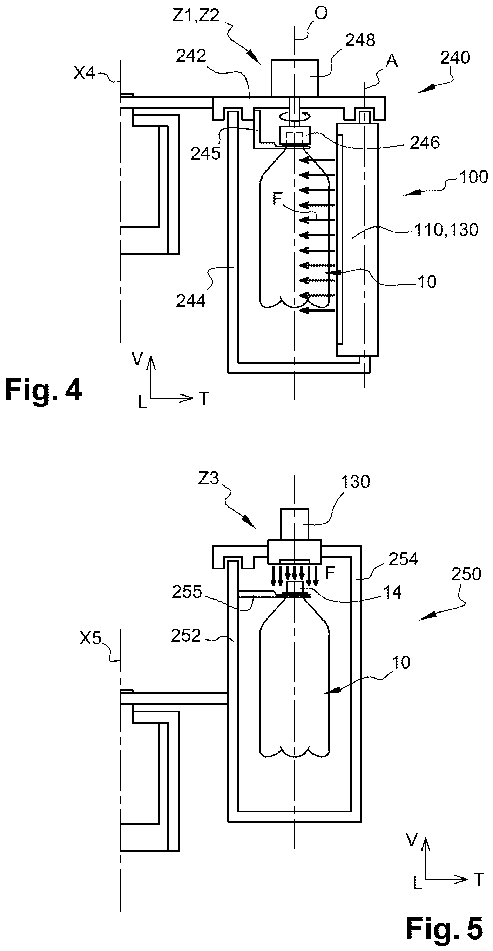

- les figures 4 et 5 sont des vues en coupe de zones d'irradiation de la figure 3 qui représentent un des récipients en cours de stérilisation depuis l'extérieur par irradiation au moyen d'un faisceau d'électrons et qui illustrent respectivement une zone d'irradiation du corps d'au moins un récipient simultanément entraîné en rotation sur lui-même et une autre zone d'irradiation du col d'au moins un récipient ;

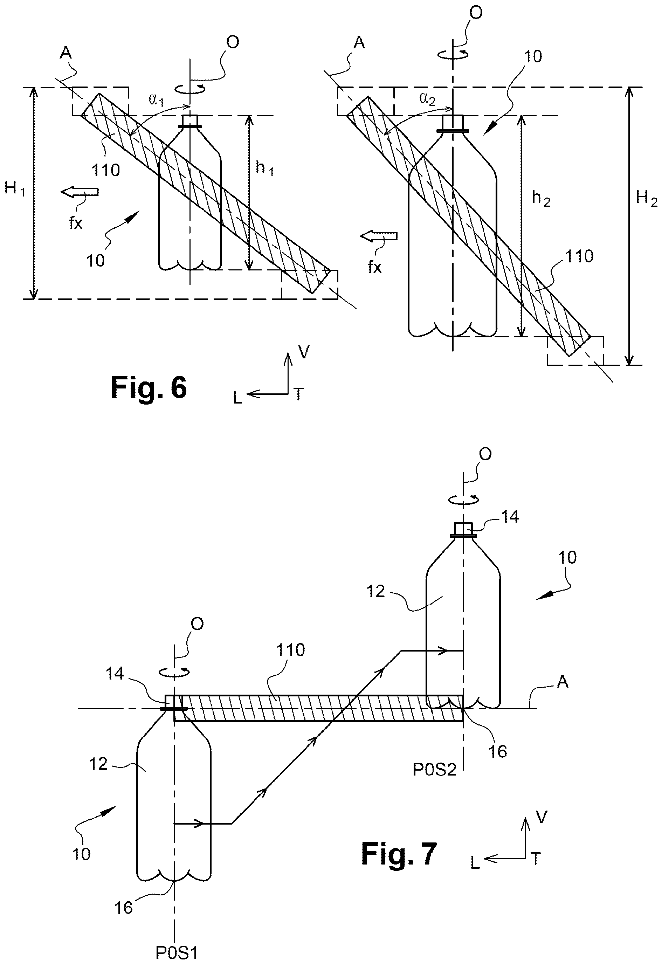

- la figure 6 est une vue de côté qui, analogue à la figure 2, représente une première variante de réalisation consistant à optimiser l'agencement des émetteurs relativement aux récipients en fonction de leurs dimensions, en particulier leur hauteur, et qui illustre pour deux récipients de dimensions différentes, une inclinaison de l'émetteur par rapport à l'axe du récipient selon un angle déterminé pour optimiser l'irradiation dudit récipient ;
- la figure 7 est une vue de côté qui représente une deuxième variante de réalisation consistant à optimiser l'irradiation des récipients relativement aux émetteurs et qui illustre un émetteur agencé horizontalement dont l'axe est orthogonal par rapport à l'axe du récipient, ledit récipient étant entraîné en rotation sur lui-même et transporté pour passer devant l'émetteur en effectuant un déplacement en oblique, suivant un mouvement de montée ou de descente, à une vitesse qui varie en fonction du type de récipient ;

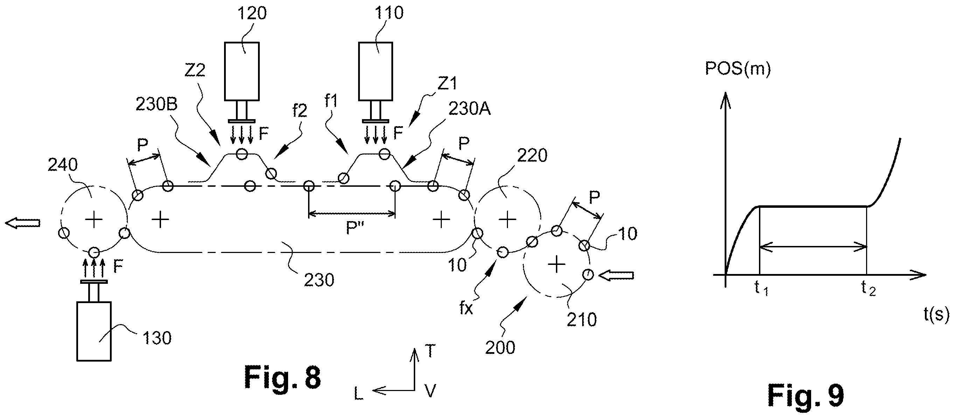

- la figure 8 est une vue de dessus qui, similaire aux figures 1 et 3, représente un flux de récipients en matière thermoplastique transportés par un système de convoyage suivant un parcours donné et qui illustre un deuxième exemple de réalisation pour la mise en oeuvre du procédé de traitement pour la stérilisation par irradiation des récipients selon l'invention dans lequel une division du flux de récipients est réalisée avant de faire varier ensuite le pas entre deux récipients consécutifs pour en augmenter la durée d'irradiation ;
- la figure 9 est une courbe qui représente, pour le deuxième exemple de réalisation selon la figure 8, la variation de la position POS (m) d'un récipient en fonction du temps t (s) et qui illustre la variation de la vitesse de chaque récipient survenant, après la division du flux de récipients, dans la première et la deuxième zones d'irradiation.

### DESCRIPTION DETAILLEE DES FIGURES

Dans la suite de la description, on adoptera à titre non limitatif les orientations longitudinale, verticale et transversale en référence au trièdre (L, V, T) représenté sur les figures.

Par convention, les orientations longitudinale et transversale définissant ensemble un plan horizontal sont déterminées de manière fixe par rapport aux récipients.

On utilisera à titre non limitatif les termes « amont » et « aval » en référence au sens de déplacement des récipients qui sont transportés par le système de convoyage de l'amont vers l'aval suivant un parcours donné.

On utilisera également à titre non limitatif les termes « supérieur » et « inférieur » ou « haut » et « bas » en référence à l'orientation verticale. Les termes « intérieur » ou « extérieur » et « interne » ou « externe » sont notamment utilisés par rapport aux récipients, le volume intérieur étant délimité par la paroi du récipient dont le corps est muni d'un col et fermé par un fond de sorte que ladite paroi délimite respectivement l'intérieur et l'extérieur.

Dans la description qui suit, les éléments désignés par les mêmes chiffres de référence désignent des moyens analogues, similaires ou identiques.

On a illustré, sur les figures 1 et 2, un dispositif 100 de stérilisation pour la mise en oeuvre d'un procédé de traitement pour la stérilisation par irradiation de récipients 10 selon l'état de la technique pour la Demanderesse.

Dans la présente demande, les récipients 10 en matière thermoplastique à stériliser sont préférentiellement des bouteilles, notamment en PET (Polyéthylène Téréphtalate), fabriquées à partir d'une préforme. En variante, le récipient 10 en matière thermoplastique est un bidon, un flacon, un pot, etc.

Tel qu'illustré sur la figure 1, le récipient 10 comporte un corps 12 muni d'un col 14 et fermé par un fond 16, ledit col 14 délimitant circonférentiellement une ouverture 18.

Le récipient 10 comporte une surface 20 interne délimitée par une paroi 22 formant le corps 12, le col 14 et le fond 16, ainsi qu'une surface 24 externe.

De préférence, le col 14 du récipient 10 comporte au moins une collerette 26 qui s'étend radialement vers l'extérieur et comporte une face 28 inférieure.

Le récipient 10 présente un axe O principal.

Tel qu'illustré sur les figures 1 et 2, l'axe O du récipient 10 s'étend ici verticalement selon le trièdre (L, V, T).

De préférence, les récipients 10 sont transportés dans une position dite « col en haut » lors de leur irradiation au défilé par au moins un faisceau (F) d'électrons délivré par le dispositif 100 de stérilisation.

Le dispositif 100 de stérilisation est par exemple intégré dans une installation (non représentée) de fabrication de récipients dans laquelle est ainsi réalisée la stérilisation par irradiation des récipients 10.

Une telle installation de fabrication de récipients 10, en particulier de bouteilles en PET, est bien connue de l'état de la technique.

La fabrication d'un récipient en matière thermoplastique y est obtenue à partir d'une préforme chaude, généralement préalablement conditionnée thermiquement dans une unité de conditionnement thermique ou four avant d'être introduite dans un des moules d'une unité de moulage (ou souffleuse) pour y être transformée par soufflage au moyen d'au moins un fluide sous pression, avec ou sans étirage.

La stérilisation des récipients est donc effectuée en aval de l'unité de soufflage ou souffleuse (non représentée) d'une telle installation de fabrication de récipients.

Dans une telle installation, les récipients 10 (et les préformes) en matière thermoplastique sont successivement transportés sous la forme d'un flux (fx) continu par un système 200 de convoyage à travers les différentes unités de l'installation assurant les étapes de fabrication.

Un tel système 200 de convoyage comporte en outre des roues de transfert dont de nombreuses réalisations sont connues.

On distingue pour mémoire principalement des roues de transfert comportant un plateau à encoches associé à des moyens de guidage et des roues de transfert comportant des pinces, commandées ou non au moins en ouverture.

La figure 1 représente ainsi un tronçon du parcours suivi par les récipients 10 provenant de l'unité de moulage, une flèche à chaque extrémité du tronçon indiquant le sens de circulation du flux (fx) de récipients 10, de l'amont vers l'aval.

Sur le tronçon du parcours de la figure 1, les récipients 10 du flux (fx) sont transportés successivement par des roues de transfert qui, ici au nombre de cinq, sont respectivement référencées 201 à 205.

Les récipients 10 sont transportés avec un écartement déterminé, dit pas P, correspondant à la distance entre les axes O de deux récipients 10 consécutifs du flux (fx).

Sur le tronçon du parcours associé au dispositif 100 de stérilisation, le pas P entre deux récipients 10 est constant, c'est-à-dire que le pas P ne varie pas entre la première roue 201 de transfert située en amont et la dernière roue 205 de transfert située en aval.

Selon une caractéristique importante, outre le pas P entre deux récipients 10 consécutifs, la vitesse de déplacement des récipients 10 formant ledit flux (fx) est ainsi constante.

La vitesse de déplacement des récipients 10 détermine la cadence de production d'une telle installation de fabrication, exprimée en nombre de récipients par heure, ladite cadence étant notamment déterminée en fonction de l'unité de remplissage.

Le dispositif 100 de stérilisation comporte des émetteurs, ici cinq émetteurs référencés successivement 101 à 105, qui sont chacun destinés à irradier lesdits récipients 10 depuis l'extérieur avec un faisceau F d'électrons.

Les émetteurs 101 à 105 sont agencés le long du parcours suivi par le flux (fx) de récipients 10, un émetteur étant par exemple associé à chacune des roues de transfert 201 à 205.

Pour la mise en oeuvre d'un procédé de traitement selon l'état de la technique, le dispositif 100 de stérilisation comporte un nombre important d'émetteurs nécessaires à l'obtention d'une dose d'irradiation finalement reçue par chaque récipient 10 qui permette d'en garantir la stérilisation.

Les émetteurs 101 à 105 sont agencés sur le côté du parcours suivi par le flux (fx) de récipients 10 de sorte que les récipients 10 sont successivement irradiés radialement depuis l'extérieur par chaque faisceau (F) d'électrons émis par l'un des émetteurs.

Chaque récipient 10 reçoit à chaque fois une dose d'irradiation donnée qui est notamment déterminée par la durée d'exposition audit faisceau (F) d'électrons, et donc par la vitesse de déplacement des récipients 10 devant les émetteurs 101 à 105.

Sur la figure 1, un récipient 10 du flux (fx) est successivement irradié par chacun des cinq émetteurs 101 à 105 du dispositif 100 de stérilisation et reçoit au final une quantité cumulée d'irradiation correspondant à une dose létale pour laquelle on obtient la stérilisation de sa surface 20 interne et de sa surface 24 externe du récipient 10.

Or, le coût d'un émetteur reste particulièrement élevé ce qui demeure un obstacle pour la stérilisation des récipients 10 par irradiation.

C'est l'une des raisons pour lesquelles, on recherche des solutions pour optimiser l'utilisation de tels émetteurs dans le but notamment de réduire le nombre total d'émetteurs utilisés, mais sans réduction des cadences de production.

Les émetteurs 101 à 105 formants ledit dispositif 100 de stérilisation sont identiques de sorte que la description de l'émetteur 101 réalisée ci-après vaut également pour les autres émetteurs du dispositif 100 de stérilisation.

Tel qu'illustré sur la figure 2, l'émetteur 101 présente un axe A principal qui s'étend verticalement selon le trièdre (L, V, T).

L'émetteur 101 présente une forme parallélépipédique et s'étend, suivant son axe A principal, sur une hauteur H. de préférence, l'émetteur 101 comporte une tête 111 qui est plus particulièrement représentée sur la figure 2.

Un émetteur 101 est ainsi susceptible d'irradier depuis l'extérieur un récipient 10 présentant verticalement une hauteur h, depuis son fond 16 jusqu'à son col 14, ladite hauteur h du récipient 10 étant inférieure ou égale à la hauteur H de l'émetteur 101.

Cependant, si la hauteur H de l'émetteur 101 est fixe, déterminée par construction, le format des récipients 10 varie lui en fonction des applications.

En effet, une même installation de fabrication est susceptible de fabriquer des récipients 10, tels que des bouteilles, allant par exemple d'une contenance d'une demi litre (ou moins) jusqu'à une contenance de deux litres.

Une partie du faisceau (F) d'électrons émis par l'émetteur 101 est donc perdue dès lors qu'aucune partie du récipient 10 ne se trouve radialement en vis-à-vis pour être irradiée.

La partie perdue du faisceau (F) d'électrons émis par un émetteur est notamment fonction de la différence entre la hauteur H et la hauteur h du récipient 10.

Cela constitue également un surcoût, au regard notamment de l'énergie électrique consommée pour alimenter l'émetteur produisant le faisceau (F) d'électrons.

On décrira ci-après, par comparaison avec l'état de la technique illustré par les figures 1 et 2, un procédé de traitement pour la stérilisation par irradiation de récipients 10 en matière thermoplastique selon l'invention.

Le récipient 10 est identique à celui décrit précédemment, c'est-à-dire du type présentant un axe O principal et comportant un corps 12 muni d'un col 14 et fermé par un fond 16.

De la même manière, le procédé de traitement selon l'invention est destiné à traiter un flux (fx) de récipients 10 transportés, de l'amont vers l'aval, par un système 200 de convoyage suivant un parcours donné, avec un écartement déterminé, dit pas P, correspondant à la distance entre les axes O de deux récipients 10 consécutifs.

Conformément à l'invention, le procédé de traitement pour la stérilisation par irradiation de récipients 10 en matière thermoplastique comporte au moins :
- une étape initiale de modification du pas consistant à faire varier ledit pas P initial entre deux récipients 10 consécutifs pour augmenter la durée d'irradiation de chaque récipient 10 ; et
- une étape d'irradiation consistant à irradier chaque récipient 10 du flux (fx) depuis l'extérieur avec un faisceau (F) d'électrons émis par ledit au moins un émetteur 110 du dispositif 100 de stérilisation.

Ledit au moins un émetteur 110 est agencé sur un tronçon dudit parcours de manière à former une zone d'irradiation des récipients 10.

Avantageusement, l'étape d'irradiation est réalisée après l'étape de modification dudit pas (P) initial.

Ainsi, l'étape d'irradiation est réalisée après l'étape de modification dudit pas (P) initial au cours de laquelle on va faire varier sélectivement le pas (P) pour pouvoir réduire la vitesse de déplacement des récipients 10 au moins dans chaque zone d'irradiation de manière à augmenter la durée d'irradiation de chaque récipient 10.

Par comparaison avec l'état de la technique, la vitesse de déplacement des récipients n'est donc pas constante le long du parcours déterminé par le système 200 de convoyage.

Toutefois, grâce notamment aux phases de décélération et d'accélération mises en oeuvre lors du changement de pas, la vitesse moyenne de déplacement reste au moins égale à celle obtenue dans l'état de la technique de sorte que les cadences de fabrication ne s'en trouvent pas modifiées, en particulier pas réduites.

Dans la présente invention, l'étape de modification du pas (P) initial est une modification temporaire dont le but est de pouvoir ensuite augmenter la durée d'irradiation de chaque récipient 10.

Outre par l'augmentation de la durée d'irradiation, l'invention propose également d'optimiser l'étape d'irradiation pour maximiser la quantité d'électrons reçue par un récipient de manière à atteindre plus rapidement la dose létale recherchée, c'est-à-dire une dose supérieure ou égale à 10 kGy (kilo-Gray).

Avantageusement, le procédé de traitement selon l'invention comporte une étape finale de modification du pas qui, par opposition à l'étape initiale de modification du pas, consiste à faire varier de nouveau l'écartement des récipients pour rétablir ledit pas (P) initial entre les récipients 10.

Avantageusement, la vitesse de déplacement de chaque récipient 10 est temporairement réduite au moins dans ladite zone d'irradiation pour augmenter la durée d'irradiation de chaque récipient 10 par ledit au moins un émetteur.

Grâce à l'augmentation de la durée d'irradiation, la dose d'irradiation émise par un émetteur 110 qui est reçue par chaque récipient 10 sera donc supérieure et ce faisant va permettre de réduire le nombre nécessaire d'émetteurs à l'obtention d'une dose létale.

Par comparaison avec l'état de la technique décrit en références aux figures 1 et 2, il est dès lors possible d'utiliser un nombre moindre d'émetteur(s), par exemple trois émetteurs contre cinq auparavant, et cela sans pour autant que la dose d'irradiation reçue par chaque récipient 10 ne s'en trouve affectée, préservant ainsi le degré de stérilisation finalement obtenu.

Avantageusement, la réduction du nombre d'émetteur(s) du dispositif 100 de stérilisation permet de réduire de manière substantielle les coûts de mise en oeuvre d'une stérilisation par irradiation au moyen d'un faisceau (F) d'électrons.

En effet, pour un résultat de stérilisation au moins équivalent, la réduction du nombre d'émetteur(s) permet tout d'abord de réduire le coût d'acquisition d'un dispositif 100 de stérilisation, notamment destiné à équiper une installation de fabrication de récipients 10.

Avantageusement, la réduction du nombre d'émetteur(s) permet ensuite de réduire aussi les coûts d'exploitation d'un dispositif 100 de stérilisation, en particulier la consommation d'énergie électrique nécessaire pour produire un faisceau (F) d'électrons.

Avantageusement, chaque récipient 10 est entraîné en rotation sur lui-même, autour de son axe O, au moins lors de l'étape d'irradiation afin que l'ensemble du récipient 10 soit irradié de manière homogène par le faisceau (F) d'électrons.

De préférence, l'étape d'irradiation pour stériliser les récipients 10 depuis l'extérieur est obtenue au moyen d'un faisceau (F) d'électrons pulsé, c'est-à-dire un faisceau d'électrons qui est formé par une succession d'impulsions.

Avantageusement, les impulsions formant ledit faisceau (F) d'électrons pulsé présentent une durée d'émission, une intensité et une énergie qui sont déterminées en fonction des applications.

On pourra par exemple se reporter aux enseignements du document WO-2016/120544 précité sur les caractéristiques physiques d'un tel faisceau (F) d'électrons de type pulsé.

On a représenté sur les figures 3 à 5, un premier exemple de réalisation pour la mise en oeuvre du procédé de traitement pour la stérilisation par irradiation de récipients selon l'invention.

Dans ce premier exemple de réalisation, le pas P initial entre deux récipients 10 consécutifs est temporairement réduit en faisant varier leur vitesse de déplacement grâce à quoi une augmentation de la durée d'irradiation est obtenue.

Dans ce premier exemple de réalisation, l'étape initiale de modification dudit pas (P) initial consiste à réduire l'écartement entre les récipients 10 dudit flux (fx) en modifiant la vitesse des récipients 10 transportés pour, au moins dans ladite zone d'irradiation, obtenir un pas P' proximal.

Le pas P' proximal obtenu est inférieur au pas P initial.

Avantageusement, l'étape initiale de modification dudit pas (P) initial comporte une phase de décélération des récipients pour obtenir ledit pas (P') proximal.

Tel qu'illustré sur la figure 3, les récipients 10 du flux (fx) sont ralentis au moins en amont de chaque zone d'irradiation grâce à quoi la durée d'irradiation de chaque récipient 10 est augmentée, augmentant la dose d'irradiation reçue par les récipients.

De préférence, le procédé de traitement comporte une étape préliminaire de détermination consistant, en fonction du diamètre D extérieur maximal d'un récipient 10, à déterminer une valeur minimale dudit pas (P') proximal.

Avantageusement, ladite valeur minimale est non nulle, ledit pas (P') proximal étant déterminé de manière à éviter tout contact entre deux récipients 10 consécutifs.

Chaque récipient 10 est ainsi apte à être entraîné en rotation sur lui-même, autour de son axe O, lors de l'étape d'irradiation, et cela sans interférence avec les récipients 10 adjacents respectivement situés en amont et en aval.

Le procédé de traitement comporte une étape finale de modification du pas consistant, après ladite au moins une étape d'irradiation, à faire varier de nouveau l'écartement des récipients 10 pour rétablir ledit pas (P) initial entre les récipients 10 dudit flux (fx).

Pour ce faire, l'étape finale de modification du pas comporte au moins une phase d'accélération.

De préférence, ladite étape d'irradiation est réalisée en dynamique sur le flux (fx) de récipients 10 qui sont transportés le long dudit parcours de manière que leur vitesse varie mais sans jamais atteindre une valeur nulle.

Avantageusement, la vitesse de déplacement des récipients 10 varie conjointement à la modification dudit pas, diminuant lors de la phase de décélération puis augmentant ensuite lors de la phase d'accélération, mais sans que le récipient 10 ne soit jamais à l'arrêt.

On décrira maintenant plus particulièrement les figures 3 à 5 illustrant un premier exemple de dispositif 100 de stérilisation pour la mise en oeuvre du procédé de traitement selon l'invention.

La figure 3 représente un tronçon du parcours suivi par le flux (fx) de récipients 10, notamment dans une installation de fabrication de récipients, ledit tronçon étant alors situé en amont de l'unité de remplissage et de préférence en aval de l'unité de soufflage.

Avantageusement, l'étape d'irradiation est réalisée sur des récipients 10 vides sortant de l'unité de soufflage qui, après leur stérilisation par irradiation au moyen d'électrons, sont ensuite remplis dans l'unité de remplissage.

Le flux (fx) de récipients 10 est convoyé par un système 200 de convoyage comportant successivement des roues de transfert, respectivement référencées 210 à 250.

La première roue 210 de transfert est alimentée en récipients 10, selon la flèche représentée sur la figure 3, provenant par exemple de l'unité de soufflage ou d'une unité d'étiquetage (non représentées) situées en amont.

Les récipients 10 sont transportés avec un pas P initial au moins par la première roue 210 de transfert, la deuxième roue 220 de transfert et la troisième roue 230 de transfert.

Tel qu'illustré sur la figure 3, le pas P initial correspond à l'écartement entre deux récipients 10 successifs, à la distance entre les axes O respectifs desdits récipients 10.

De préférence, le dispositif 100 de stérilisation comporte au moins un premier émetteur 110 et un deuxième émetteur 120 qui sont agencés pour irradier les récipients 10 depuis l'extérieur, plus particulièrement pour irradier au moins le corps 12 et le fond 16 de chaque récipient 10.

Le premier émetteur 110 et le deuxième émetteur 120 sont ici associés à la quatrième roue 240 de transfert du flux (fx) de récipients 10.

Le premier émetteur 110 et le deuxième émetteur 120 sont agencés radialement, ici à l'extérieur, de la quatrième roue 240 de transfert, sur le côté du parcours suivi par les récipients 10.

Le premier émetteur 110 et le deuxième émetteur 120 sont agencés chacun sur un tronçon du parcours des récipients 10 de manière à former une première zone Z1 d'irradiation et une deuxième zone Z2 d'irradiation.

Le premier émetteur 110 et le deuxième émetteur 120 sont destinés à la mise en oeuvre d'une première étape d'irradiation que comporte ladite étape d'irradiation du procédé de traitement.

Avantageusement, ladite étape d'irradiation comporte une première étape d'irradiation consistant à irradier depuis l'extérieur au moins le corps 12 de chaque récipient 10 avec le faisceau (F) d'électrons émis par ledit au moins un émetteur, ici successivement par le premier émetteur 110 et le deuxième émetteur 120.

Le premier émetteur 110 et le deuxième émetteur 120, présentant respectivement un axe (A) principal, sont agencés relativement audit parcours des récipients 10 de manière que ledit axe (A) principal de chaque émetteur soit sensiblement coaxial avec l'axe (O) principal du récipient lors de l'irradiation.

Avantageusement, chaque récipient 10 est entrainé en rotation sur lui-même autour de son axe O lors de ladite première étape d'irradiation de manière à irradier circonférentiellement l'ensemble du récipient 10.

Avantageusement, le dispositif 100 de stérilisation comporte également un troisième émetteur 130 qui est ici associé à la cinquième roue 250 de transfert.

Le troisième émetteur 130 est agencé sur un tronçon du parcours des récipients 10 de manière à former une troisième zone Z3 d'irradiation.

Le troisième émetteur 130 est agencé pour irradier les récipients 10 depuis l'extérieur, plus particulièrement pour irradier au moins le col 14 de chaque récipient 10.

De préférence, le troisième émetteur 130 est agencé à l'aplomb, ici au-dessus, du parcours suivi par le flux (fx) de récipients 10 transportés par la cinquième roue 250 de transfert du système 200 de convoyage.

Avantageusement, ladite étape d'irradiation du procédé comporte une deuxième étape d'irradiation consistant à irradier au moins le col 14 de chaque récipient 10 avec un faisceau (F) d'électrons émis par au moins un autre émetteur, ici ledit troisième émetteur 130.

Le troisième émetteur 130, présentant un axe (A) principal, est agencé relativement audit parcours de manière que ledit axe (A) principal de l'émetteur soit sensiblement orthogonal à l'axe (O) principal du récipient 10 irradié.

Chaque récipient 10 est avantageusement immobile en rotation lors de ladite deuxième étape d'irradiation.

On a représenté sur la figure 4 une vue en coupe réalisée au niveau du deuxième émetteur 120 selon un plan IV-IV illustré sur la figure 3, ledit plan s'étendant verticalement et radialement en passant par l'axe (X4) de rotation de la quatrième roue 240 de transfert.

Le premier émetteur 110 et le deuxième émetteur 120 sont identiques de sorte que la description de la figure 4 donnée ci-après en référence au deuxième émetteur 120 vaut également pour le premier émetteur 110.

De préférence, la quatrième roue 240 de transfert comporte des moyens de protection qui, associés au moins au premier émetteur 110 et au deuxième émetteur 120, entourent les récipients 10 pour former une enceinte de confinement.

Avantageusement, les moyens de protection comportent des moyens 242 mobiles de protection qui sont entraînés en rotation autour de l'axe (X4) de rotation et des moyens 244 fixes de protection qui sont immobiles relativement au flux (fx) de récipients 10.

De préférence, la partie du premier émetteur 110 et la partie du deuxième émetteur 120 émettant respectivement un faisceau (F) d'électrons sont intégrées auxdits moyens 244 fixes de protection.

Dans la quatrième roue 240 de transfert, les récipients 10 sont transportés par l'intermédiaire de moyens 245 de préhension qui, reliés à des bras radiaux, sont entraînés autour de l'axe X5 de rotation.

De préférence, les moyens 245 de préhension comportent des pinces destinées à coopérer chacune avec une partie du col 14 du récipient 10 et qui sont aptes à être commandées sélectivement en ouverture.

La quatrième roue 240 de transfert comporte des moyens 246 d'entraînement en rotation de chaque récipient 10 sur lui-même, autour de son axe O principal.

Les moyens 246 d'entraînement en rotation comportent un élément d'entraînement configuré pour coopérer avec une partie du col 14 de chaque récipient 10, par exemple un mandrin qui est logé dans l'ouverture 18 du col 14.

Les moyens 246 d'entraînement sont aptes à être entraînés en rotation par des moyens 248 d'actionnement, tels qu'un moteur.

De préférence, les moyens 245 de préhension sont commandés en ouverture pour ne pas serrer radialement le col 14 du récipient 10 afin de permettre librement leur entraînement en rotation par les moyens 246 d'entraînement

Tel qu'illustré sur la figure 3, la vitesse de chaque récipient 10 du flux (fx) est ralentie au moins en amont de la première zone Z1 d'irradiation de manière à en modifier le pas, à réduire l'écartement entre deux récipients 10 consécutifs.

Avantageusement, on augmente ainsi la durée d'irradiation de chaque récipient 10 dès lors que le temps d'exposition du récipient 10 au faisceau (F) d'électrons émis par l'émetteur est augmenté.

La phase de décélération permet alors de passer du pas P initial à un pas P' proximal qui est inférieur audit pas P initial. C'est la raison pour laquelle, les récipients 10 sont regroupés, rapprochés les uns des autres, devant le premier émetteur 110 et ensuite devant le deuxième émetteur 120.

L'étape initiale de modification du pas permet d'augmenter la durée d'irradiation des récipients 10 lorsque les récipients 10 traversent la première zone Z1 d'irradiation avec une vitesse inférieure à leur vitesse moyenne de déplacement.

Avantageusement, le ralentissement consécutif à la phase de décélération est ensuite compensé par au moins une phase d'accélération équivalente, conformément à l'étape finale de modification du pas, de manière à rétablir ledit pas P initial.

La variation de pas entre les récipients 10 est par exemple réalisée à l'aide de moyens de commande de type mécaniques, notamment à came et à galet.

En variante, les moyens de commande de la variation de pas sont électriques, par exemple un moteur préférentiellement un moteur linéaire.

La même variation de pas, de pas (P) initial au pas (P') proximal, est réalisée dans la deuxième zone Z2 d'irradiation, les récipients 10 subissant successivement une phase de décélération pour augmenter la durée d'irradiation par le faisceau (F) d'électrons puis une phase d'accélération.

De préférence, la phase de décélération est principalement réalisée hors desdites première zone Z1 d'irradiation et deuxième zone Z2 d'irradiation mais peut s'y poursuivre.

De même, la phase d'accélération est principalement réalisée hors desdites première zone Z1 d'irradiation et deuxième zone Z2 d'irradiation mais peut y débuter.

L'irradiation des récipients 10 est également augmentée par la modification de l'écartement des récipients 10 qui sont juxtaposés les uns à côté des autres lorsque l'écartement est égal au pas P' proximal.

En effet et par comparaison avec la figure 2 illustrant l'état de la technique, le faisceau (F) d'électrons ne rencontre alors pas d'espace vide entre deux récipients 10 consécutifs grâce à quoi l'utilisation des électrons produits par un émetteur est optimisée.

Le système 200 de convoyage est configuré pour faire varier le pas des récipients 10 sur ladite quatrième 240 roue de transfert de manière que la vitesse moyenne de déplacement des récipients 10 reste inchangée.

Avantageusement, le flux (fx) de récipients 10 présente à la sortie de la quatrième 240 roue de transfert un écartement entre deux récipients 10 consécutifs correspondant au pas (P) initial.

De préférence, l'irradiation du col 14 du récipient 10 d'une part et du corps 12 d'autre part sont réalisées successivement et non simultanément.

L'irradiation des cols 14 est réalisée sur la cinquième roue 250 de transfert par l'intermédiaire du troisième émetteur 130 du dispositif 100 de stérilisation.

Comme la quatrième roue 240, la cinquième roue 250 de transfert comporte préférentiellement des moyens de protection, respectivement des moyens 252 mobiles de protection qui sont entraînés en rotation autour de l'axe (X5) de rotation et des moyens 254 fixes de protection qui sont immobiles relativement au flux (fx) de récipients 10 transportés.

De préférence, la partie du troisième émetteur 130 émettant le faisceau (F) d'électrons est intégrée à une partie supérieure desdits moyens 254 fixes de protection.

Sur la quatrième roue 240 de transfert, l'entraînement en rotation du récipient 10 étant réalisé par le col 14, les moyens 246 d'entraînement sont alors susceptibles d'en masquer une partie au faisceau (F) d'électrons de sorte qu'il existe un risque que le col 14 bien qu'irradié ne soit pas parfaitement stérilisé.

C'est la raison pour laquelle, une irradiation spécifique du col 14 de chaque récipient 10 est mise en oeuvre.

Avantageusement, l'irradiation du col 14 est réalisée après celle du corps 12. Ainsi, même en cas de contamination du col 14 par exemple par les moyens 246 d'entraînement en rotation, l'irradiation postérieure du col 14 permet de garantir une parfaite stérilisation du récipient 10.

Lors de l'irradiation du col 14, le récipient 10 est maintenu en position « col en haut » par l'intermédiaire de moyens 255 de préhension qui sont configurés pour laisser le col 14 dégagé, libre d'être irradié par le faisceau (F) d'électrons émis par le troisième émetteur 130.

Par comparaison avec l'étape d'irradiation du récipient 10 réalisée sur la quatrième roue 240 de transfert par le premier émetteur 110 et le deuxième émetteur 120, le récipient 10 n'est pas entraîné en rotation sur lui-même lors de l'irradiation du col 14 réalisée par le troisième émetteur 130 sur la cinquième roue.

De préférence, l'étape d'irradiation des cols 14 est réalisée sur une autre roue de transfert que celle des corps 12 et sur des récipients 10 présentant ledit pas P initial.

Bien entendu, la description qui vient d'être faite de l'irradiation successive du corps 12 puis du col 14 de chaque récipient 10 ne doit pas être interprétée limitativement mais comme visant plus particulièrement une partie du récipient 10 dès lors que le faisceau (F) d'électrons émis par chacun des émetteurs 110, 120 et 130 irradie l'ensemble du récipient 10.

Dans le premier exemple illustré aux figures 3 à 5, les émetteurs 110 et 120 sont agencés de manière que l'axe A principal de l'émetteur s'étend verticalement, coaxialement à l'axe O de chacun des récipients 10.

Or, tel qu'expliqué en préambule en référence à la figure 2, un émetteur présente une hauteur H déterminée par construction tandis que la hauteur h des récipients 10 fabriqués varie quant à elle en fonction des applications.

Ainsi, lorsque le récipient 10 présente une hauteur h qui est la moitié de la hauteur H de l'émetteur, la moitié du faisceau (F) d'électrons émis par l'émetteur est alors perdue puisque cette partie du faisceau (F) ne va irradier aucun récipient.

Pour y remédier, l'invention propose deux variantes d'agencement d'un émetteur permettant d'améliorer encore la stérilisation obtenue en augmentant, pour une durée d'irradiation équivalente, la dose d'irradiation reçue par chaque récipient 10.

Par comparaison à l'état de la technique, une telle augmentation de la dose d'électrons reçue par le récipient 10 participe avantageusement à la réduction du nombre d'émetteur(s) en exploitant de manière optimale le faisceau (F) d'électrons émis.

La figure 6 représente une première variante de réalisation consistant à optimiser l'agencement dudit au moins un émetteur, tels que le premier émetteur 110 et le deuxième émetteur 120, relativement au récipient 10 en fonction de ses dimensions, en particulier sa hauteur h.

On a représenté sur la figure 6 deux récipients 10 de dimensions différentes, un premier récipient 10 présentant une hauteur h1 et un deuxième récipient 10 présentant une hauteur h2, supérieure à la hauteur h1 du premier récipient.

Pour optimiser l'irradiation d'un récipient 10 et limiter les pertes d'électrons émis par un émetteur, l'émetteur est incliné relativement au récipient en fonction de la hauteur du récipient 10 de manière que le ratio de la hauteur H du récipient sur la hauteur h de l'émetteur soit proche de 1.

On détermine ainsi un angle d'inclinaison correspondant à l'angle (α) formé par l'intersection de l'axe A principal de l'émetteur et l'axe O du récipient, la direction de déplacement suivi par le récipient qui est illustrée par une flèche sur la figure 6 étant orthogonale à l'axe O principal du récipient.

Avantageusement, l'émetteur est configuré pour incliner de l'angle (α) une tête que comporte ledit émetteur, ladite tête formant la partie terminale émettant ledit faisceau (F) d'électrons irradiant au moins un récipient 10.

Tel qu'illustré sur la figure 6, l'émetteur 110 est par exemple incliné suivant un angle (α1) d'inclinaison compris entre son axe A principal et l'axe O du récipient 10 de hauteur (H1) ou incliné suivant un angle (α2) d'inclinaison compris entre son axe A principal et l'axe O du récipient 10 de hauteur (H2).

L'angle (α) d'inclinaison de l'émetteur varie ainsi en fonction du récipient 10 à irradier, la valeur dudit angle (α) étant déterminé pour optimiser l'irradiation dudit récipient par le faisceau (F) d'électrons, pour maximiser la dose d'irradiation reçue.

De préférence, l'émetteur 110 est incliné de manière que les deux extrémités s'étendent respectivement au-delà du col 14 et du fond 16 du récipient 10, lesdites extrémités de l'émetteur 110 ayant été représentées sur la figure 6 par des rectangles en trait mixte.

Avantageusement, le col 14 et le fond 16 du récipient 10 sont ainsi irradiés de manière optimale par le faisceau (F) d'électrons émis par la partie de l'émetteur 110 se trouvant radialement en vis-à-vis.

Avantageusement, le récipient 10 est entraîné en rotation sur lui-même, autour de son axe O, pendant son irradiation par le faisceau (F) d'électrons émis par l'émetteur 110.

Avantageusement, le procédé de traitement selon l'invention comporte au moins une étape préliminaire de réglage consistant à positionner ledit au moins un émetteur présentant un axe A principal suivant un angle (α) d'inclinaison qui, compris entre l'axe A principal de l'émetteur et l'axe O du récipient, est déterminé en fonction de la hauteur (H) du récipient transporté suivant une direction de déplacement qui est orthogonale à l'axe O principal du récipient.

La figure 7 représente une deuxième variante de réalisation pour optimiser la dose d'irradiation en jouant sur l'agencement relatif entre un émetteur, tel que le premier émetteur 110, et le flux (fx) de récipients 10.

Tel qu'illustré sur la figure 7, l'émetteur 110 est agencé horizontalement selon le trièdre (L, V, T) de sorte que l'axe A principal de l'émetteur est orthogonal par rapport à l'axe O du récipient 10, chaque récipient 10 étant comme précédemment transporté en position dite « col en haut ».

Avantageusement, le récipient 10 est entraîné en rotation sur lui-même, autour de son axe O, pendant son irradiation par le faisceau (F) d'électrons émis par l'émetteur 110.

Les moyens d'entraînement en rotation comme les moyens de préhension du récipient 10 n'ont pas été représentés sur la figure 7 mais sont par exemple analogues à ceux décrits en référence aux figures 3 à 5.

La figure 7 représente à gauche un récipient 10 dans une première position POS1, dite basse, dans laquelle le col 14 dudit récipient 10 se trouve en vis-à-vis de l'émetteur 110.

La figure 7 représente à droite ce récipient 10 dans une deuxième position POS2, dite haute, dans laquelle le fond 16 dudit récipient 10 se trouve en vis-à-vis de l'émetteur 110 pour être irradier par le faisceau (F) d'électrons émis par ledit émetteur 110.

Avantageusement, la première position POS1, dite basse, permet d'irradier plus particulièrement le col 14 tandis que la deuxième position POS2, dite haute, permet d'irradier plus particulièrement le fond 16.

En effet, le col 14 et/ou le fond 16 sont des parties très sensibles, parfois difficiles à stériliser, par exemple en raison de l'épaisseur de matière thermoplastique ou de la forme pétaloïde pour un fond 16.

Entre la première position POS1, dite basse, et la deuxième position POS2, dite haute, le récipient 10 est transporté pour passer devant l'émetteur 110 en effectuant un déplacement comportant au moins une portion de trajectoire oblique dans la zone d'irradiation.

Selon le sens du déplacement, le récipient 10 effectue soit un mouvement de montée de la première position POS1, dite basse, vers la deuxième position POS2, dite haute, soit inversement un mouvement de descente de la deuxième position POS2, dite haute, vers la première position POS1, dite basse.

De préférence, la vitesse de chaque récipient 10 n'est pas constante sur ladite portion de trajectoire oblique comprise entre lesdites première position POS1, dite basse, et deuxième position POS2, dite haute.

Avantageusement, la vitesse du récipient 10 varie donc dans la zone d'irradiation comprise entre lesdites première position POS1, dite basse, et deuxième position POS2, dite haute.

La vitesse du récipient 10 peut en particulier être réduite pour augmenter sélectivement la durée d'irradiation d'une partie du récipient 10.

A titre d'exemple, la vitesse du récipient 10 est minimale lorsque le récipient 10 se trouve dans lesdites première position POS1, dite basse, et/ou deuxième position POS2, dite haute, afin d'augmenter la durée d'irradiation du col 14 et/ou du fond 16 du récipient 10.

De préférence, l'étape d'irradiation des récipients 10 est réalisée de manière dynamique sur un récipient 10 toujours en mouvement, que la vitesse varie ou soit constante.

Bien entendu, la vitesse du récipient 10 pourrait être aussi temporairement nulle, chaque récipient marquant par exemple un arrêt dans lesdites première position POS1, dite basse, et/ou deuxième position POS2, dite haute.

On a représenté sur la figure 7 uniquement deux récipients 10 pour illustrer lesdites première position POS1, dite basse, et deuxième position POS2, dite haute, toutefois et selon l'invention une étape initiale de modification du pas est avantageusement réalisée avant l'étape d'irradiation.

Selon le premier exemple décrit en référence aux figures 3 à 5, les récipients 10 du flux (fx) se présentent par exemple dans la zone d'irradiation un pas (P') proximal qui est inférieur au pas (P) initial.

Avantageusement, le procédé de traitement selon l'invention comporte au moins une étape préliminaire de paramétrage consistant, ledit au moins un émetteur présentant un axe A principal étant agencé horizontalement avec un angle (α) d'inclinaison de 90° entre l'axe A principal de l'émetteur et l'axe O du récipient, à paramétrer au moins la vitesse de déplacement du flux (fx) de récipients dans la zone d'irradiation.

Avantageusement, outre la vitesse de déplacement, la vitesse d'entraînement en rotation sur lui-même de chaque récipient 10 est également un paramètre qui est déterminé en fonction des applications.

Le parcours des récipients 10 dans la zone d'irradiation s'effectue selon une direction qui, n'étant pas orthogonale à l'axe O principal des récipients, correspond à un mouvement de monte ou baisse pour optimiser l'irradiation des récipients.

Par comparaison avec la variante de la figure 6, l'émetteur 110 occupe dans cette variante une position horizontale fixe qui reste la même quelle que soit les dimensions du récipient 10, notamment la hauteur h du récipient.

Dans cette variante selon la figure 7, l'optimisation de l'irradiation du récipient 10 est obtenue par un paramétrage de la vitesse de déplacement du récipient dans la zone d'irradiation.

Pour chaque récipient 10, il est ainsi possible d'optimiser l'étape d'irradiation pour faire varier sélectivement la durée d'irradiation en fonction du type de récipient 10, pour augmenter par exemple la dose reçue par le col 14 et/ou le fond 16 du récipient 10 ou encore une partie spécifique du corps 12.

On a représenté sur les figures 8 et 9, un deuxième exemple de réalisation pour la mise en oeuvre du procédé de traitement pour la stérilisation par irradiation de récipients selon l'invention.

Dans ce deuxième exemple de réalisation et conformément à l'invention, l'étape d'irradiation des récipients 10 est mise en oeuvre après l'étape initiale de modification du pas.

Selon ce deuxième exemple, le procédé de traitement comporte une étape de division du flux (fx), mise en oeuvre préalablement à l'étape de modification dudit pas (P) initial, consistant à diviser le flux (fx) de récipients 10 transportés.

On a représenté sur la figure 8, comme précédemment pour la figure 1 ou 3, un dispositif 100 de stérilisation.

Grâce à l'invention et comme dans le premier exemple, le nombre d'émetteurs du dispositif 100 de stérilisation est avantageusement réduit à trois émetteurs contre cinq émetteurs dans l'état de la technique selon les figures 1 et 2.

Le dispositif 100 de stérilisation comporte ainsi un premier émetteur 110 et un deuxième émetteur 120, respectivement destinés à irradier au moins le corps 12 des récipients 10, et un troisième émetteur 130 destiné à irradier au moins le col 14 des récipients.

Par comparaison, le flux (fx) de récipients 10 est transporté par un système 200 de convoyage comportant une première roue 210 de transfert, une deuxième roue 220 de transfert et une quatrième roue 240 de transfert.

Dans ce deuxième exemple, la troisième « roue » du système 200 de convoyage est modifiée et réalisée sous la forme d'un dispositif 230 de transfert présentant une forme de piste allongée.

Le dispositif 230 de transfert comporte un tronçon de parcours globalement rectiligne, alimenté en amont par la deuxième roue 220 et alimentant en aval la quatrième roue 240.

Le dispositif 230 de transfert comporte sur ledit tronçon rectiligne, un premier tronçon 230A de dérivation et un deuxième tronçon 230B de dérivation, auxquels sont respectivement associés le premier émetteur 110 et le deuxième émetteur 120.

Tel qu'illustré sur la figure 8, le premier émetteur 110 agencé sur le côté du premier tronçon 230A de dérivation forme une première zone Z1 d'irradiation tandis que le deuxième émetteur 120 agencé sur le côté du deuxième tronçon 230B de dérivation forme une deuxième zone Z2 d'irradiation.

La structure et le fonctionnement du troisième émetteur 130 associé à la quatrième roue 240 de transfert et plus particulièrement destiné à irradier le col 14 des récipients 10 est analogue à la description donnée pour le premier exemple à laquelle on se reportera avantageusement.

Les récipients 10 circulent de l'amont vers l'aval selon les flèches représentées sur la figure 8, les récipients 10 du flux (fx) transportés présentant sur la première roue 210 de transfert, la deuxième roue 220 de transfert et la quatrième roue 240 de transfert, un écartement correspondant au pas (P) initial.

L'étape de division du flux (fx) de récipients 10 est réalisée sur le dispositif 230 de transfert.

Avantageusement, le flux (fx) de récipients 10 est divisé en au moins :
- un premier flux (f1) de récipients qui sont transportés vers la première zone Z1 d'irradiation comportant au moins le premier émetteur 110, et
- un deuxième flux (f2) de récipients qui sont transportés vers la deuxième zone (Z2) d'irradiation comportant au moins un deuxième émetteur 120.

De préférence, le flux (fx) de récipients est divisé à raison d'un récipient 10 sur deux pour former respectivement ledit premier flux (f1) et ledit deuxième flux (f2).

Après l'étape de division, l'écartement entre deux récipients 10 consécutifs de l'un ou l'autre desdits premier et deuxième flux (f1, f2) est alors égal à un pas (P") distal qui correspondant au double du pas (P) initial.

Par comparaison avec le premier exemple, à l'issue de l'étape de division, les récipients 10 ne sont pas rapprochés les uns des autres mais au contraire plus éloignés.

Grâce à cette augmentation de l'écartement ou pas entre deux récipients 10 consécutifs de chacun desdits premier et deuxième flux (f1, f2), il est alors possible de faire varier de manière plus importante la vitesse de chaque récipient 10 et cela sans risquer d'interférence avec les récipients 10 situés immédiatement en amont et en aval.

Après la division du flux (fx) de récipients, l'étape initiale de modification du pas est avantageusement mise en oeuvre.

L'étape initiale de modification du pas comporte au moins une phase de décélération pour réduire la vitesse de chacun des récipients 10 constituant l'un desdits au moins premier flux (f1) et deuxième flux (f2) au moins dans lesdites première zone Z1 d'irradiation et deuxième zone Z2 d'irradiation associées.

Chaque récipient 10 passe ainsi devant un des émetteurs 110 ou 120 avec une vitesse réduite, voire avantageusement nulle, de manière à augmenter la durée d'irradiation de chaque récipient 10 et par conséquent la dose d'électrons reçue.

De préférence, le récipient 10 est temporairement arrêté devant l'émetteur de la première zone Z1 d'irradiation comme celui de la deuxième zone Z2 d'irradiation, c'est-à-dire que sa vitesse est momentanément nulle.

Grâce à la réduction importante de la vitesse, voir l'arrêt temporaire du récipient 10, il est possible d'irradier au moins le corps 12 de chaque récipient 10 en ayant recours qu'à un seul émetteur, le premier émetteur 110 et le deuxième émetteur 120 étant ici disposé en parallèle.

Par comparaison, dans le premier exemple, le corps 12 de chaque récipient 10 est irradié deux fois, successivement par le premier émetteur 110 puis par le deuxième émetteur 120, ledit deuxième émetteur 120 étant disposé en série avec le premier émetteur 110.

L'étape de modification du pas comporte également, au moins une phase d'accélération pour augmenter la vitesse de chacun des récipients 10 desdits premier et deuxième flux (f1, f2) afin, de conserver la même vitesse moyenne de convoyage des récipients 10.

Avantageusement et comme précédemment, les cadences de fabrication dans le cas d'une installation ne sont donc pas impactées par les variations de vitesse réalisées dans le but d'augmenter la durée d'irradiation de chaque récipient 10.

Avantageusement, une phase d'accélération est effectuée respectivement en amont et en aval de l'émetteur dans chacune desdites première et deuxième zone Z1, Z2 d'irradiation pour pouvoir encore maximiser la durée d'irradiation.

On a représenté sur la figure 9 une courbe pour illustrer la variation de la vitesse subie par un récipient 10 circulant dans l'un des tronçons 230A, 230B de dérivation du dispositif 230 de transfert.

La courbe de la figure 9 représente la distance parcourue POS (m) par un récipient 10 en fonction du temps t(s).

Tel qu'illustré par la première partie de ladite courbe, le récipient 10 subit tout d'abord une première phase d'accélération pendant une durée t1.

Sur la partie médiane de la courbe, on peut voir une phase de décélération qui s'achève avec l'arrêt du récipient 10 devant l'émetteur, entre les points t1 et t2 le récipient 10 ne parcourt en effet aucune distance supplémentaire.

Lorsque la durée d'irradiation du récipient 10 correspondant à (t2-t1) est terminée, le récipient 10 subit alors à nouveau une phase d'accélération.

Le récipient 10 irradié poursuit son parcours sur celui des tronçons 230A, 230B de dérivation sur lequel il se trouvait jusqu'à rejoindre le tronçon principal du dispositif 230 de transfert où le premier flux (f1) et deuxième flux (f2) sont regroupés pour reformer le flux (fx) de récipients 10.

Avantageusement, le procédé comporte une étape finale de modification du pas consistant à regrouper le premier flux (f1) et le deuxième flux (f2) pour reformer un flux (fx) de récipients 10 présentant à nouveau ledit pas (P) initial.

Pour un dispositif 100 de stérilisation selon la figure 8, le système 200 de convoyage est avantageusement un système utilisant des navettes auxquelles sont associées des moteurs linéaires.

A titre d'exemple non limitatif, un système de convoyage du type « acopos trak » développé par la société B&R / industrial automation est susceptible d'être utilisé pour assurer la division du flux que les variations de vitesse en vue de l'irradiation, individuellement pour chaque récipient transporté.

Bien entendu, l'une ou l'autre des variantes de réalisation décrites précédemment en référence aux figures 6 et 7 pour maximiser la dose d'irradiation reçue par un récipient 10 sur une durée donnée est avantageusement susceptible d'être intégrée à un dispositif 100 de stérilisation selon la figure 8 pour la mise en oeuvre du deuxième exemple de réalisation du procédé de traitement selon l'invention.

L'invention est susceptible d'application industrielle et propose un procédé de traitement pour la stérilisation par irradiation de récipients 10 en matière thermoplastique, comportant au moins :
- une étape initiale de modification du pas consistant à faire varier sélectivement le pas P initial entre deux récipients 10 consécutifs pour augmenter la durée d'irradiation de chaque récipient 10 ;
- une étape d'irradiation consistant à irradier chaque récipient 10 depuis l'extérieur avec un faisceau F d'électrons émis par au moins un émetteur 110, 120, 130 d'un dispositif 100 de stérilisation.

## Revendications

1. Procédé de traitement pour la stérilisation par irradiation de récipients (10) en matière thermoplastique présentant un axe (O) principal et comportant un corps (12) muni d'un col (14) et fermé par un fond (16), dans lequel les récipients (10) formant un flux (fx) sont transportés par un système (200) de convoyage suivant un parcours donné, avec un écartement déterminé, dit pas (P), correspondant à la distance entre les axes (O) de deux récipients (10) consécutifs, et la vitesse de déplacement des récipients (10) formant ledit flux (fx) est constante, le procédé de traitement comportant au moins :
- une étape initiale de modification du pas consistant à faire varier sélectivement ledit pas (P) initial entre deux récipients (10) consécutifs pour augmenter la durée d'irradiation de chaque récipient (10) ;
- une étape d'irradiation consistant à irradier chaque récipient (10) du flux (fx) depuis l'extérieur avec un faisceau (F) d'électrons émis par au moins un émetteur (110, 120, 130) d'un dispositif (100) de stérilisation qui est agencé sur un tronçon dudit parcours de manière à former une zone (Z1, Z2, Z3) d'irradiation des récipients (10).

2. Procédé de traitement selon la revendication 1, **caractérisé en ce que** l'étape initiale de modification dudit pas (P) initial consiste à réduire l'écartement entre les récipients (10) dudit flux (fx) en réduisant la vitesse des récipients (10) transportés pour obtenir, au moins dans ladite zone d'irradiation, un pas (P') proximal qui est inférieur au pas (P) initial.

3. Procédé de traitement selon la revendication 2, **caractérisé en ce que** l'étape initiale de modification dudit pas (P) initial comporte au moins une phase de décélération des récipients pour obtenir ledit pas (P') proximal.

4. Procédé de traitement selon l'une des revendications 2 à 3, **caractérisé en ce que** ledit procédé comporte
- une étape finale de modification du pas comportant au moins une phase d'accélération pour faire varier à nouveau l'écartement des récipients (10) afin de rétablir ledit pas (P) initial entre les récipients (10) dudit flux.

5. Procédé de traitement selon la revendication 1, **caractérisé en ce que** ledit procédé comporte une étape de division du flux (fx), mise en oeuvre préalablement à l'étape initiale de modification du pas, consistant à diviser le flux (fx) de récipients (10) transportés, en au moins :
- un premier flux (f1) de récipients qui sont transportés vers une première zone (Z1) d'irradiation comportant au moins un premier émetteur (110) apte à émettre un faisceau (F) d'électrons et
- un deuxième flux (f2) de récipients qui sont transportés vers une deuxième zone (Z2) d'irradiation comportant au moins un deuxième émetteur (120) apte à émettre un faisceau (F) d'électrons.

6. Procédé de traitement selon la revendication 5, **caractérisé en ce que** le flux (fx) de récipients est divisé à raison d'un récipient (10) sur deux pour former respectivement ledit premier flux (f1) et ledit deuxième flux (f2) de sorte que l'écartement entre deux récipients (10) consécutifs de l'un ou l'autre desdits premier flux (f1) et deuxième flux (f2) est alors égal à un pas (P") distal qui est supérieur au pas (P) initial.

7. Procédé de traitement selon l'une des revendications 5 ou 6, **caractérisé en ce que**, après la division du flux (fx) de récipients, ladite étape initiale de modification du pas (P) initial comporte au moins une phase de décélération pour réduire la vitesse de chacun des récipients (10) constituant l'un desdits au moins premier flux (f1) et deuxième flux (f2) au moins dans lesdites première zone (Z1) d'irradiation et deuxième zone (Z2) d'irradiation associées.

8. Procédé de traitement selon la revendication 7, **caractérisé en ce que**, après l'étape de division du flux (fx) de récipients, ladite étape de modification du pas (P) initial comporte au moins une phase d'accélération pour augmenter sélectivement la vitesse de chacun des récipients (10) desdits premier flux (f1) et deuxième flux (f2), respectivement en amont et/ou en aval desdites première zone (Z1) et deuxième zone (Z2) d'irradiation.

9. Procédé de traitement selon l'une des revendications 4 à 8, **caractérisé en ce que** ledit procédé comporte :
- une étape finale de modification du pas consistant, après ladite au moins une étape d'irradiation, à fusionner ensemble les récipients (10) desdits premier flux (f1) et deuxième flux (f2) pour obtenir un flux (fx) de récipients (10) présentant ledit pas (P) initial entre deux récipients (10) consécutifs.

10. Procédé de traitement selon l'une quelconque des revendications précédentes, **caractérisé en ce que** ladite étape d'irradiation comporte une première étape d'irradiation consistant à irradier depuis l'extérieur au moins le corps de chaque récipient (10) avec le faisceau (F) d'électrons émis par ledit au moins un émetteur (110, 120) qui présentant un axe (A) principal.

11. Procédé de traitement selon la revendication 10, **caractérisé en ce que**, lorsque la direction de déplacement suivi par le flux (fx) de récipients (10) est orthogonale à l'axe (O) principal des récipients (10), ledit au moins un émetteur (110, 120) est agencé relativement audit parcours de manière que ledit axe (A) principal de l'émetteur soit coaxial avec l'axe (O) principal du récipient (10) irradié.

12. Procédé de traitement selon la revendication 10, **caractérisé en ce que**, lorsque la direction de déplacement suivi par le flux (fx) de récipients (10) comporte au moins un tronçon incliné relativement à l'axe (A) dudit au moins émetteur (110, 120) de sorte que chaque récipient (10) effectue un déplacement en oblique suivant un mouvement de montée ou de descente, ledit au moins un émetteur (110, 120) est agencé horizontalement pour présenter un angle (α) d'inclinaison de 90° entre son axe (A) principal et l'axe (O) d'un récipient.

13. Procédé de traitement selon l'une quelconque des revendications précédentes, **caractérisé en ce que** chaque récipient (10) est entrainé en rotation sur lui-même autour de son axe (O) lors au moins de ladite première étape d'irradiation.

14. Procédé de traitement selon la revendication 10, **caractérisé en ce que** ladite étape d'irradiation comporte une deuxième étape d'irradiation consistant à irradier au moins le col (14) de chaque récipient (10) avec un faisceau (F) d'électrons émis par ledit au moins un émetteur (130) qui, présentant un axe (A) principal, est agencé à l'aplomb des récipients (10) suivant ledit parcours.

## Patentansprüche

1. Bearbeitungsverfahren für die Sterilisation von Behältern (10) aus thermoplastischem Material durch Bestrahlung, die eine Hauptachse (O) haben und einen Körper (2) aufweisen, der mit einem Kragen (14) versehen ist und von einem Boden (16) verschlossen wird, wobei die einen Strom (fx) bildenden Behälter (10) von einem Beförderungssystem (200) gemäß einer gegebenen Strecke transportiert werden, mit einem bestimmten Abstand, Teilung (P) genannt, der der Entfernung zwischen den Achsen (O) von zwei aufeinanderfolgenden Behältern (10) entspricht, und die Verschiebegeschwindigkeit der den Strom (fx) bildenden Behälter (10) konstant ist, wobei das Bearbeitungsverfahren mindestens aufweist:
- einen Initialschritt der Änderung der Teilung, der darin besteht, die Initialteilung (P) zwischen zwei aufeinanderfolgenden Behältern (10) selektiv zu variieren, um die Bestrahlungsdauer jedes Behälters (10) zu erhöhen;
- einen Schritt der Bestrahlung, der darin besteht, jeden Behälter (10) des Stroms (fx) von außen mit einem von mindestens einem Emitter (110, 120, 130) einer Sterilisationsvorrichtung (100) emittierten Elektronenstrahl zu bestrahlen, die auf einem Abschnitt der Strecke eingerichtet ist, um einen Bestrahlungsbereich (Z1, Z2, Z3) der Behälter (10) zu bilden.

2. Bearbeitungsverfahren nach Anspruch 1, **dadurch gekennzeichnet, dass** der Initialschritt der Änderung der Initialteilung (P) darin besteht, den Abstand zwischen den Behältern (10) des Stroms (fx) durch Verringerung der Geschwindigkeit der transportierten Behälter (10) zu verringern, um mindestens im Bestrahlungsbereich eine proximale Teilung (P') zu erhalten, die geringer ist als die Initialteilung (P).

3. Bearbeitungsverfahren nach Anspruch 2, **dadurch gekennzeichnet, dass** der Initialschritt der Änderung der Initialteilung (P) mindestens eine Phase der Verlangsamung der Behälter aufweist, um die proximale Teilung (P') zu erhalten.

4. Bearbeitungsverfahren nach einem der Ansprüche 2 bis 3, **dadurch gekennzeichnet, dass** das Verfahren aufweist
- einen Finalschritt der Änderung der Teilung, der mindestens eine Beschleunigungsphase aufweist, um den Abstand der Behälter (10) erneut zu variieren, um die Initialteilung (P) zwischen den Behältern (10) des Stroms wiederherzustellen.

5. Bearbeitungsverfahren nach Anspruch 1, **dadurch gekennzeichnet, dass** das Verfahren einen vor dem Initialschritt der Änderung der Teilung durchgeführten Schritt der Unterteilung des Stroms (fx) enthält, der darin besteht, den Strom (fx) von transportierten Behältern (10) zu unterteilen in mindestens:
- einen ersten Strom (f1) von Behältern, die zu einem ersten Bestrahlungsbereich (Z1) transportiert werden, der mindestens einen ersten Emitter (110) aufweist, der fähig ist, einen Elektronenstrahl (F) zu emittieren, und
- einen zweiten Strom (f2) von Behältern, die zu einem zweiten Bestrahlungsbereich (Z2) transportiert werden, der mindestens einen zweiten Emitter (120) aufweist, der fähig ist, einen Elektronenstrahl (F) zu emittieren.

6. Bearbeitungsverfahren nach Anspruch 5, **dadurch gekennzeichnet, dass** der Strom (fx) von Behältern für jeden zweiten Behälter (10) unterteilt wird, um den ersten Strom (f1) bzw. den zweiten Strom (f2) zu bilden, so dass der Abstand zwischen zwei aufeinanderfolgenden Behältern (10) des einen oder anderen der ersten (f1) und zweiten Ströme (f2) dann gleich einer distalen Teilung (P") ist, die größer ist als die Initialteilung (P).

7. Bearbeitungsverfahren nach einem der Ansprüche 5 oder 6, **dadurch gekennzeichnet, dass** nach der Unterteilung des Stroms (fx) von Behältern der Initialschritt der Änderung der Initialteilung (P) mindestens eine Verlangsamungsphase aufweist, um die Geschwindigkeit jedes der einen der mindestens ersten (f1) und zweiten Ströme (f2) bildenden Behälter (10) mindestens in den verbundenen ersten (Z1) und zweiten Bestrahlungsbereichen (Z2) zu verringern.

8. Bearbeitungsverfahren nach Anspruch 7, **dadurch gekennzeichnet, dass** nach dem Schritt der Unterteilung des Stroms (fx) von Behältern der Schritt der Änderung der Initialteilung (P) mindestens eine Beschleunigungsphase aufweist, um die Geschwindigkeit jedes der Behälter (10) des ersten Stroms (f1) und des zweiten Stroms (f2) stromaufwärts vor und/oder stromabwärts hinter den ersten (Z1) und zweiten Bestrahlungsbereichen (Z2) selektiv zu erhöhen.

9. Bearbeitungsverfahren nach einem der Ansprüche 4 bis 8, **dadurch gekennzeichnet, dass** das Verfahren aufweist:
- einen Finalschritt der Änderung der Teilung, der nach dem mindestens einen Bestrahlungsschritt darin besteht, die Behälter (10) des ersten Stroms (f1) und des zweiten Stroms (f2) miteinander zu vereinen, um einen Strom (fx) von Behältern (10) zu erhalten, der die Initialteilung (P) zwischen zwei aufeinanderfolgenden Behältern (10) aufweist.

10. Bearbeitungsverfahren nach einem der vorhergehenden Ansprüche, **dadurch gekennzeichnet, dass** der Bestrahlungsschritt einen ersten Bestrahlungsschritt aufweist, der darin besteht, von außen mindestens den Körper jedes Behälters (10) mit dem vom mindestens einen eine Hauptachse (A) aufweisenden Emitter (110, 120) emittierten Elektronenstrahl (F) zu bestrahlen.

11. Bearbeitungsverfahren nach Anspruch 10, **dadurch gekennzeichnet, dass**, wenn die vom Strom (fx) von Behältern (10) durchlaufene Verschieberichtung orthogonal zur Hauptachse (O) der Behälter (10) ist, der mindestens eine Emitter (110, 120) bezüglich der Strecke so angeordnet ist, dass die Hauptachse (A) des Emitters mit der Hauptachse (O) des bestrahlten Behälters (10) koaxial ist.

12. Bearbeitungsverfahren nach Anspruch 10, **dadurch gekennzeichnet, dass**, wenn die vom Strom (fx) von Behältern (10) durchlaufene Verschieberichtung mindestens einen bezüglich der Achse (A) des mindestens einen Emitters (110, 120) geneigten Abschnitt aufweist, so dass jeder Behälter (10) eine schräge Verschiebung gemäß einer Anstiegs- oder Abstiegsbewegung ausführt, der mindestens eine Emitter (110, 120) waagrecht angeordnet ist, um einen Neigungswinkel (α) von 90° zwischen seiner Hauptachse (A) und der Achse (O) eines Behälters aufzuweisen.

13. Bearbeitungsverfahren nach einem der vorhergehenden Ansprüche, **dadurch gekennzeichnet, dass** jeder Behälter (10) mindestens im ersten Bestrahlungsschritt um seine Achse (O) um sich selbst in Drehung versetzt wird.

14. Bearbeitungsverfahren nach Anspruch 10, **dadurch gekennzeichnet, dass** der Bestrahlungsschritt einen zweiten Bestrahlungsschritt aufweist, der darin besteht, mindestens den Hals (14) jedes Behälters (10) mit einem Elektronenstrahl (F) zu bestrahlen, der vom mindestens einem Emitter (130) emittiert wird, der, indem er eine Hauptachse (A) aufweist, lotrecht vor den Behältern (10) entlang der Strecke angeordnet ist.

## Claims

1. Treatment method for the sterilization by irradiation of containers (10) made of thermoplastic material having a main axis (O) and comprising a body (12) provided with a neck (14) and closed by a bottom (16), wherein the containers (10) forming a flow (fx) are transported by a conveying system (200) along a given path, with a determined separation, called pitch (P), corresponding to the distance between the axes (O) of two consecutive containers (10), and the speed of displacement of the containers (10) forming said flow (fx) is constant, the treatment method comprising at least:
- an initial step of modification of the pitch consisting in making said initial pitch (P) between two consecutive containers (10) vary selectively to increase the irradiation time of each container (10);
- an irradiation step consisting in irradiating each container (10) of the flow (fx) from the outside with an electron beam (F) emitted by at least one emitter (110, 120, 130) of a sterilization device (100) which is arranged on a section of said path so as to form a zone (Z1, Z2, Z3) of irradiation of the containers (10).

2. Treatment method according to Claim 1, **characterized in that** the initial step of modification of said initial pitch (P) consists in reducing the separation between the containers (10) of said flow (fx) by reducing the speed of the containers (10) transported to obtain, at least in said irradiation zone, a proximal pitch (P') which is less than the initial pitch (P).

3. Treatment method according to Claim 2, **characterized in that** the initial step of modification of said initial pitch (P) comprises at least one phase of deceleration of the containers to obtain said proximal pitch (P').

4. Treatment method according to one of Claims 2 and 3, **characterized in that** said method comprises
- a final step of modification of the pitch comprising at least one acceleration phase to once again vary the separation of the containers (10) in order to re-establish said initial pitch (P) between the containers (10) of said flow.

5. Treatment method according to Claim 1, **characterized in that** said method comprises a step of division of the flow (fx), implemented prior to the initial step of modification of the pitch, consisting in dividing the flow (fx) of transported containers (10), into at least:
- a first flow (f1) of containers which are transported to a first irradiation zone (Z1) comprising at least one first emitter (110) capable of emitting an electron beam (F) and
- a second flow (f2) of containers which are transported to a second irradiation zone (Z2) comprising at least one second emitter (120) capable of emitting an electron beam (F).

6. Treatment method according to Claim 5, **characterized in that** the flow (fx) of containers is divided at a rate of one container (10) in every two to respectively form said first flow (f1) and said second flow (f2) such that the separation between two consecutive containers (10) of one or the other of said first flow (f1) and second flow (f2) is then equal to a distal pitch (P") which is greater than the initial pitch (P).

7. Treatment method according to one of Claims 5 and 6, **characterized in that**, after the division of the flow (fx) of containers, said initial step of modification of the initial pitch (P) comprises at least one deceleration phase to reduce the speed of each of the containers (10) forming one of said at least first flow (f1) and second flow (f2) at least in said associated first irradiation zone (Z1) and second irradiation zone (Z2).

8. Treatment method according to Claim 7, **characterized in that**, after the step of division of the flow (fx) of containers, said step of modification of the initial pitch (P) comprises at least one acceleration phase to selectively increase the speed of each of the containers (10) of said first flow (f1) and second flow (f2), respectively upstream and/or downstream of said first irradiation zone (Z1) and second irradiation zone (Z2).

9. Treatment method according to one of Claims 4 to 8, **characterized in that** said method comprises:
- a final step of modification of the pitch consisting, after said at least one irradiation step, in merging together the containers (10) of said first flow (f1) and second flow (f2) to obtain a flow (fx) of containers (10) having said initial pitch (P) between two consecutive containers (10).

10. Treatment method according to any one of the preceding claims, **characterized in that** said irradiation step comprises a first irradiation step consisting in irradiating from the outside at least the body of each container (10) with the electron beam (F) emitted by said at least one emitter (110, 120) having a main axis (A).

11. Treatment method according to Claim 10, **characterized in that**, when the direction of displacement followed by the flow (fx) of containers (10) is orthogonal to the main axis (O) of the containers (10), said at least one emitter (110, 120) is arranged relative to said path in such a way that said main axis (A) of the emitter is coaxial with the main axis (O) of the irradiated container (10).

12. Treatment method according to Claim 10, **characterized in that**, when the direction of displacement followed by the flow (fx) of containers (10) comprises at least one section that is inclined relative to the axis (A) of said at least one emitter (110, 120) such that each container (10) performs an oblique displacement by a raising or lowering movement, said at least one emitter (110, 120) is arranged horizontally to exhibit an angle (α) of inclination of 90° between its main axis (A) and the axis (O) of a container.

13. Treatment method according to any one of the preceding claims, **characterized in that** each container (10) is driven in rotation on itself about its axis (O) in at least said first irradiation step.

14. Treatment method according to Claim 10, **characterized in that** said irradiation step comprises a second irradiation step consisting in irradiating at least the neck (14) of each container (10) with an electron beam (F) emitted by said at least one emitter (130) which, having a main axis (A), is arranged plumb with the containers (10) following said path.
